# EUROPEAN PATENT SPECIFICATION

(11) **EP 3 430 990 B1**
(45) Date of publication and mention of the grant of the patent: **14.02.2024**
(21) Application number: 17789379.9
(22) Date of filing: 19.04.2017
(51) Int. Cl.: A61B 5/02, A61B 5/022, A61B 5/021, A61B 5/0225, A61B 5/00

(54) **PULSE WAVE DETECTION DEVICE AND BIOLOGICAL INFORMATION MEASUREMENT DEVICE**
PULSWELLENDETEKTIONSVORRICHTUNG UND VORRICHTUNG ZUR MESSUNG BIOLOGISCHER INFORMATIONEN
DISPOSITIF DE DÉTECTION D'ONDES DE POULS ET DISPOSITIF DE MESURE D'INFORMATIONS BIOLOGIQUES

(30) Priority: 27.04.2016 JP 2016089789
(43) Date of publication of application: 23.01.2019
(73) Proprietor: Omron Healthcare Co., Ltd., Muko-shi, Kyoto 617-0002 (JP)
(72) Inventor: YAMASHITA, Shingo, Muko-shi Kyoto 617-0002 (JP); OGURA, Toshihiko, Muko-shi Kyoto 617-0002 (JP)
(74) Representative: Isarpatent
(86) International application number: PCT/JP2017/015753
(87) International publication number: WO 2017/188094

(56) References cited:
- EP-A1- 3 430 987
- WO-A1-2016/040256
- JP-A- H01 288 228
- JP-A- 2002 330 932
- JP-A- 2002 330 932
- JP-A- 2004 243 104
- JP-U- H0 520 709
- JP-U- H0 520 709

## Description

### Technical Field

The present invention relates to a pulse wave detector and a biometric information measurement device.

### Background Art

A biometric information measurement device has been known which can measure biometric information such as a pulse, a heartbeat, a blood pressure and the like by using information detected by a pressure sensor in a state where the pressure sensor is in contact with a body surface of a living body part through which the artery such as the radius artery of the wrist passes (refer to Patent Literatures 1 and 2).

Patent Literature 1 discloses a biometric information measurement device including a pressure sensor having a plurality of element arrays consisting of a plurality of pressure detection elements, a pressing part configured to press the pressure sensor, a rotation mechanism for rotating the pressure sensor about an axis extending in a direction (a direction perpendicular to the artery) perpendicular to an arrangement direction of the plurality of element arrays, and a drive unit configured to drive the rotation mechanism.

Patent Literature 2 discloses a biometric information measurement device including a rotation mechanism for rotating a pressure sensor about an axis in a direction along the artery.

### Citation List

### Patent Literature

Patent Literature 1: JP-A-H01-288228
Patent Literature 2: JP-A-H06-507563
JPH0520709 U describes a pulse wave detecting device for detecting a pressure pulse wave.
EP 3 430 987 A1 describes a pulse wave detection device which flexibly changes a pressing state towards a body surface.
JP 2002 330932 A describes a detector with a group of pressure sensitive elements and the group is pressurized to an artery.

### Summary of Invention

### Technical Problem

The body surface and the artery are not necessarily parallel with each other. A position of the artery in a depth direction is different depending on individuals. Since the biometric information measurement device disclosed in Patent Literature 1 is configured to rotate the pressure sensor about the axis extending in the direction perpendicular to the artery, it is possible to set a pressing surface of the pressure sensor to be parallel with the artery in conformity to the position of the artery in the depth direction, so that it is possible to improve detection accuracy of a pulse wave.

In order to further improve the detection accuracy of the pulse wave, it is preferable to increase a rotation amount of the pressure sensor about the axis extending in the direction perpendicular to the artery. However, the biometric information measurement device disclosed in Patent Literature 1 has a configuration where the pressing part, the rotation mechanism and the pressure sensor are arranged in this order. For this reason, a distance between a rotary axis of the rotation mechanism and the pressing surface of the pressure sensor is small, so that there is a limit to increase the rotation amount of the pressure sensor.

Also, hard tissues such as bone, tendon or the like exist in the vicinity of the artery, and a pressure signal that is to be detected by the plurality of element arrays may include many signals corresponding to pressures from the hard tissues. In order to detect the pulse wave to occur in the artery with high accuracy, it is preferable to detect the pressure signal by the plurality of element arrays while excluding an influence of the pressures from the hard tissue such as bone, tendon or the like as much as possible. However, Patent Literature 1 does not consider the influence of the pressures from the hard tissue such as bone, tendon or the like.

Also, when pressing the pressure sensor to the living body part, a position of the artery may be changed by a pressing force. Patent Literature 1 does not consider following to the position change of the artery.

The biometric information measurement device disclosed in Patent Literature 2 can rotate the pressure sensor about the axis extending in the direction along the artery. However, the rotation of the pressure sensor is performed so as to release a pressure to be applied from the wrist and the rotation is not electrically performed.

That is, the biometric information measurement device disclosed in Patent Literature 2 cannot electrically control a contact manner of the pressure sensor to the artery and is difficult to sufficiently follow the position change of the artery. The biometric information measurement device disclosed in Patent Literature 2 has a configuration where the pressing part, the rotation mechanism and the pressure sensor are arranged in this order. For this reason, as described above, there is a limit to increase the rotation amount of the pressure sensor, and it is difficult to sufficiently follow the position change of the artery.

The present invention has been made in view of the above circumstances. It is an object of the present invention to provide a pulse wave detector and a biometric information measurement device which can improve detection accuracy of a pulse wave by flexibly changing a pressing state to a body surface of a sensor unit to be used with being pressed to the body surface.

### Solution to Problem

A pulse wave detector according to the present invention includes: a sensor unit which includes a plurality of element arrays, each including pressure detecting elements arranged in one direction, the element arrays being arranged in a direction perpendicular to the one direction; a pressing part which is configured to press the sensor unit to a body surface of a living body in a state where the one direction intersects with an extension direction of an artery under the body surface; a rotation mechanism which is configured to rotate the sensor unit about a first axis extending in the one direction; and a rotation drive unit which is configured to drive the rotation mechanism, wherein the rotation mechanism, the pressing part and the sensor unit are arranged in this order in a pressing direction of the pressing part.

A pulse wave detector according to the present invention includes: a sensor unit which includes an element array including a plurality of pressure detection elements arranged in one direction; a pressing part which is configured to press the sensor unit to a body surface of a living body in a state where the one direction intersects with an extension direction of an artery under the body surface; a rotation mechanism which is configured to rotate the sensor unit about a second axis extending in a direction perpendicular to each of a pressing direction of the pressing part and the one direction; and a rotation drive unit which is configured to drive the rotation mechanism, wherein the rotation mechanism, the pressing part and the sensor unit are arranged in this order in the pressing direction.

A biometric information measurement device according to the present invention includes: the above pulse wave detector; and a biometric information calculation unit which is configured to calculate biometric information based on pulse waves detected by the pressure detection elements of the sensor unit.

### Advantageous Effects of Invention

According to the present invention, it is possible to provide the pulse wave detector and the biometric information measurement device which can improve detection accuracy of the pulse wave by flexibly changing a pressing state to a body surface of the sensor unit to be used with being pressed to the body surface.

### Brief Description of Drawings

Fig. 1 is a schematic view illustrating an outer configuration of a pulse wave detection unit 100 of a biometric information measurement device according to an embodiment of the present invention.
Fig. 2 illustrates the pulse wave detection unit 100 in an attached state of Fig. 1, as seen from an elbow side of a measurement subject.
Fig. 3 illustrates the pulse wave detection unit 100 in the attached state of Fig. 1, as seen from a contact part with a wrist.
Fig. 4 is a block diagram of parts of the biometric information measurement device of the embodiment except the pulse wave detection unit 100.
Figs. 5A to 5C are schematic views for illustrating operations of the biometric information measurement device of the embodiment.
Figs. 6A and 6B are schematic views for illustrating operations of the biometric information measurement device of the embodiment.

### Description of Embodiment

Hereinafter, embodiments of the present invention will be described with reference to the drawings.

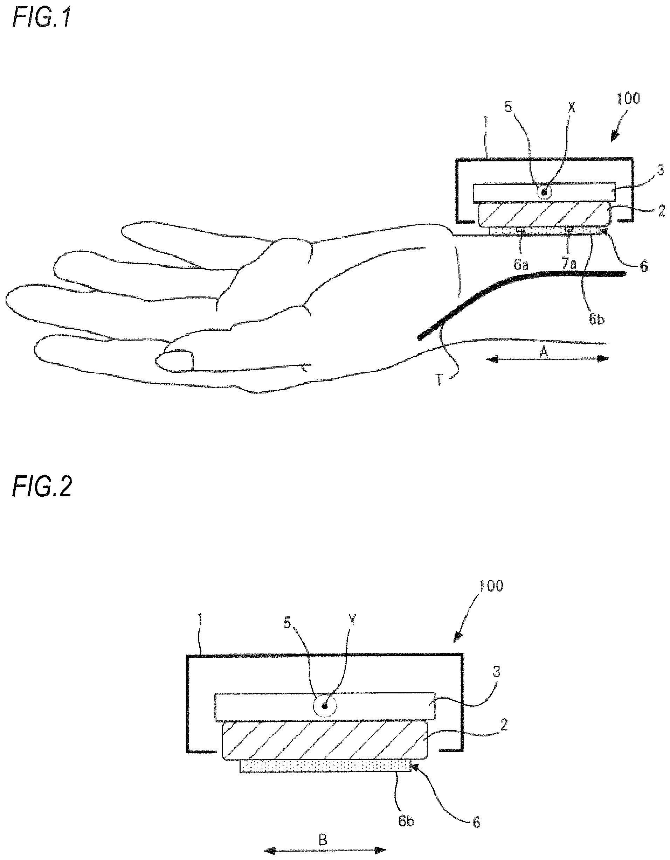

Fig. 1 is a schematic view illustrating an outer configuration of a pulse wave detection unit 100 of a biometric information measurement device according to an embodiment of the present invention. The biometric information measurement device of the embodiment is used with being attached to a living body part (a wrist of a left hand of a user, in the example of Fig. 1), in which the artery (the radius artery T, in the example of Fig. 1) that is a biometric information measurement target exists, by a band (not shown).

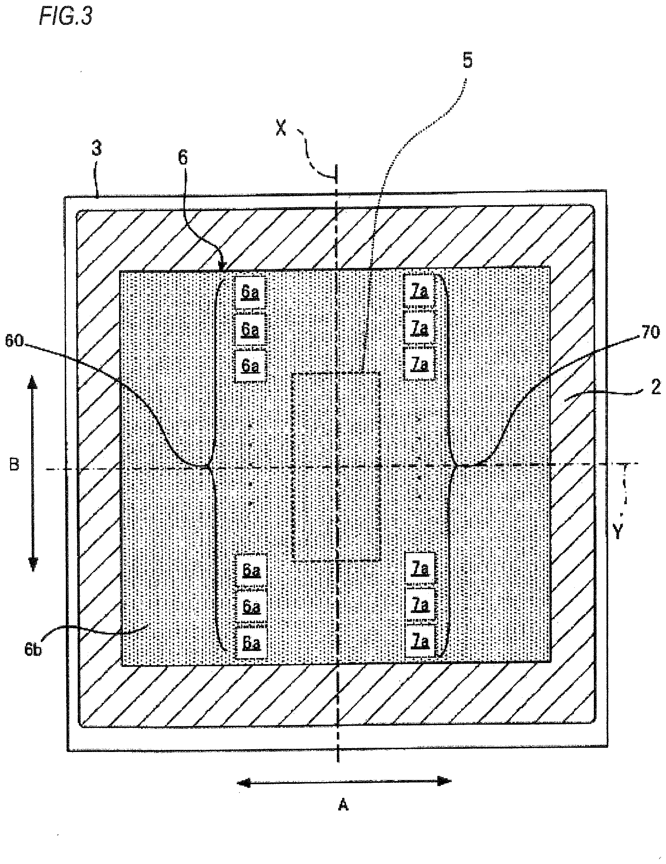

Fig. 2 illustrates the pulse wave detection unit 100 in the attached state of Fig. 1, as seen from an elbow side of a measurement subject. Fig. 3 illustrates the pulse wave detection unit 100 in the attached state of Fig. 1, as seen from a contact part with the wrist. Figs. 1 to 3 illustrate the pulse wave detection unit 100 only schematically, and do not limit sizes, arrangement and the like of respective parts.

The pulse wave detection unit 100 includes an air bag 2, a flat plate-shaped support member 3 configured to support the air bag 2, a rotation mechanism 5 for rotating the support member 3, and a sensor unit 6 fixed to the air bag 2. The air bag 2, the support member 3, the rotation mechanism 5 and the sensor unit 6 are supported by a housing 1 having an opening formed therein.

An air amount in the air bag 2 is controlled by a pump (not shown), so that the sensor unit 6 fixed to the air bag 2 is moved in a direction perpendicular to an opening surface of the housing 1.

As shown in Fig. 1, the air bag 2 functions as a pressing part configured to press a pressing surface 6b of the sensor unit 6 to a body surface of a living body part (wrist) in a state where the pulse wave detection unit 100 is attached to the wrist. As the pressing part, any mechanism capable of pressing the sensor unit 6 in a direction perpendicular to the pressing surface 6b can be used. That is, the pressing part is not limited to one using the air bag.

In the attached state of Fig. 1, the pressing surface 6b of the sensor unit 6 included in the pulse wave detection unit 100 is in contact with skin of the wrist of the user. In this state, the air amount to be introduced into the air bag 2 increases, so that an internal pressure of the air bag 2 increases and the sensor unit 6 is pressed toward the body surface. In the below, it is described that a pressing force to the body surface by the sensor unit 6 is equivalent to the internal pressure of the air bag 2.

As shown in Fig. 3, the sensor unit 6 includes an element array 60 having a plurality of pressure detection elements 6a arranged in a direction B, which is one direction, and an element array 70 having a plurality of pressure detection elements 7a arranged in the direction B. The element array 60 and the element array 70 are arranged in a direction A perpendicular to the direction B. In the state where the pulse wave detection unit 100 is attached to the wrist, the element array 60 is arranged at the periphery side and the element array 70 is arranged at the central side.

Each pressure detection element 6a and the pressure detection element 7a, which is located at the same position as the pressure detection element 6a in the direction B, configure a pair, and a plurality of pairs are aligned in the direction B in the sensor unit 6. As the pressure detection element 6a and the pressure detection element 7a, a strain gauge resistance type element, a semiconductor piezo resistance type element, an electrostatic capacitance type element or the like is used, respectively.

The respective pressure detection elements included in the element array 60 and the element array 70 are formed on the same plane, and the plane is protected by a protection member such as resin. The plane on which the respective pressure detection elements are formed and a surface of the protection member for protecting the plane are parallel with each other, and the surface of the protection member configures a pressing surface 6b.

The respective pressure detection elements 6a (7a) are pressed to the radius artery T so that the alignment direction thereof intersects (is substantially perpendicular to) with the radius artery T. Accordingly, it is possible to detect a pressure oscillation wave, i.e., a pulse wave, which is to be generated from the radius artery T and to be transmitted to the skin.

An interval of the pressure detection elements 6a (7a) in the alignment direction is sufficiently small so that the necessary number of the pressure detection elements are to be arranged above the radius artery T. An arrangement length of the pressure detection elements 6a (7a) is set to be sufficiently larger than a diameter of the radius artery T.

The rotation mechanism 5 is a mechanism for rotating the sensor unit 6 (the pressing surface 6b) about each of a first axis X and a second axis Y, which are two axes perpendicular to a pressing direction of the sensor unit 6 by the air bag 2.

The rotation mechanism 5 is configured to be rotatively driven by a rotation drive unit 10 (described later) to rotate the support member 3 about each of the first axis X and the second axis Y

The first axis X is an axis extending in the arrangement direction (direction B) of the pressure detection elements of the element array 60 or the element array 70. In the example of Fig. 3, the first axis X is set between the element array 60 and the element array 70 (at a middle part, in the example of Fig. 3). A position of the first axis X in the direction A is arbitrary.

The second axis Y is an axis extending in the arrangement direction (direction A) of the element array 60 and the element array 70. In the example of Fig. 3, the second axis Y is set on a line by which the element array 60 and the element array 70 are equally divided, respectively. A position of the second axis Y in the direction B is arbitrary.

When the support member 3 is rotated about the first axis X, the air bag 2 supported by the support member 3 and the sensor unit 6 fixed to the air bag 2 are rotated about the first axis X. Also, when the support member 3 is rotated about the second axis Y, the air bag 2 supported by the support member 3 and the sensor unit 6 fixed to the air bag 2 are rotated about the second axis Y

In the below, the rotation of the sensor unit 6 about the first axis X is referred to as pitch rotation. Also, the rotation of the sensor unit 6 about the second axis Y is referred to as roll rotation.

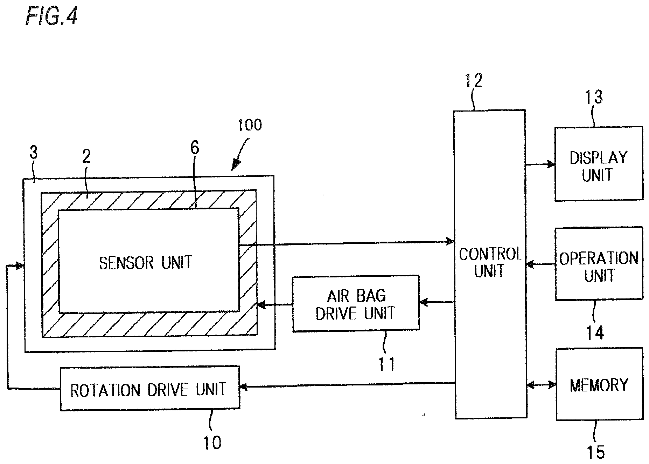

Fig. 4 is a block diagram of parts except the pulse wave detection unit 100 of the biometric information measurement device of the embodiment.

The biometric information measurement device includes the pulse wave detection unit 100, a rotation drive unit 10, an air bag drive unit 11, a control unit 12 configured to collectively control the entire device, a display unit 13, an operation unit 14, and a memory 15.

The rotation drive unit 10 is an actuator configured to drive the rotation mechanism 5 of the pulse wave detection unit 100. The rotation drive unit 10 is configured to drive the rotation mechanism 5 in accordance with an instruction of the control unit 12, thereby rotating the sensor unit 6 about the first axis X or rotating the sensor unit 6 about the second axis Y.

The air bag drive unit 11 includes a pump and the like, and is configured to control an air amount (internal pressure of the air bag 2) to be supplied to the air bag 2, under control of the control unit 12.

The display unit 13 displays various information such as biometric information, and is configured by a liquid crystal display device, for example.

The operation unit 14 is an interface for inputting an instruction signal to the control unit 12, and is configured by buttons for instructing starts of diverse operations including measurement of the biometric information, and the like.

The memory 15 is a storage medium in which a pressure signal (pulse wave) detected by the sensor unit 6 and to be used for calculation of the biometric information and various information such as the calculated biometric information are stored, and is configured by a flash memory and the like, for example. The memory 15 may be a detachable memory.

The control unit 12 is configured mainly by a processor, and includes a ROM (Read Only Memory) in which a program to be executed by the processor is stored, a RAM (Random Access Memory) functioning as a work memory, and the like.

The control unit 12 has following functions as the processor executes the program.

The control unit 12 is configured to control the air bag drive unit 11 to regulate the air amount in the air bag 2 so as to control the pressing force of the sensor unit 6 to the wrist.

The control unit 12 is configured to control the rotation drive unit 10 to rotate the support member 3, thereby controlling a rotating angle of the sensor unit 6.

The control unit 12 is configured to control the rotating angle of the sensor unit 6 to an arbitrary value, and to store a pressure signal (pulse wave), which is to be detected by the pressure detection element selected from the sensor unit 6 in a state (hereinafter, referred to as 'pulse wave measurement state') where the sensor unit 6 is pressed to the body surface by the air bag 2, in the memory 15.

The control unit 12 is configured to calculate biometric information based on the pressure signal detected in the pulse wave measurement state and stored in the memory 15, and to store the calculated biometric information in the memory 15. The control unit 12 functions as the biometric information calculation unit.

As the biometric information, any information that can be calculated based on the pulse wave may be used. For example, the control unit 12 is configured to calculate, as the biometric information, blood pressure information such as SBP (Systolic Blood pressure) and DBP (Diastolic Blood pressure), pulse information such as the number of pulses, heartbeat information such as the number of heartbeats, and the like.

In the meantime, the biometric information calculation unit may be provided to an electronic device separate from the biometric information measurement device. In this case, the pressure signal stored in the memory 15 of the biometric information measurement device is transmitted to the electronic device, and the biometric information is calculated and stored in the electronic device.

The pulse wave detector is configured by the pulse wave detection unit 100, the rotation drive unit 10, the air bag drive unit 11 and the control unit 12.

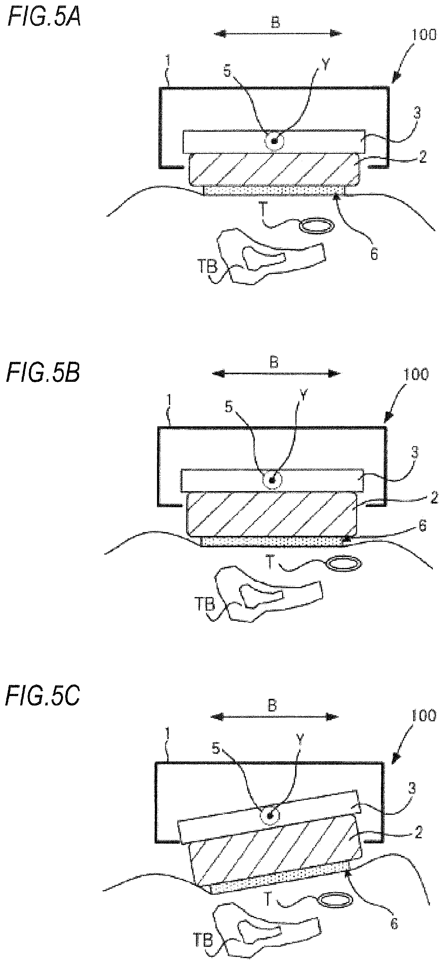

Figs. 5A to 5C are schematic views for illustrating operations of the biometric information measurement device of the embodiment. Figs. 5A to 5C illustrate the pulse wave detection unit 100 attached to a left wrist of a measurement subject, as seen from a left elbow side of the measurement subject. In Figs. 5A to 5C, the radius TB is shown.

In the biometric information measurement device configured as described above, as shown in Figs. 5A and 5B, the control unit 12 gradually increases the internal pressure of the air bag 2, so that the sensor unit 6 is pressed to the body surface.

When the sensor unit 6 is pressed to the body surface, the radius artery T may be moved in the direction B by the pressing force, as shown in Fig. 5B. In this case, the control unit 12 rotates the support member 3 about the second axis Y in a counterclockwise direction, as shown in Fig. 5C. Thereby, the pressing surface 6b of the sensor unit 6 is rotated about the second axis Y in the counterclockwise direction, so that the radius artery T is positioned immediately below the element arrays 60, 70 of the sensor unit 6. Therefore, it is possible to increase the number of the pressure detection elements capable of detecting the pulse wave with high accuracy.

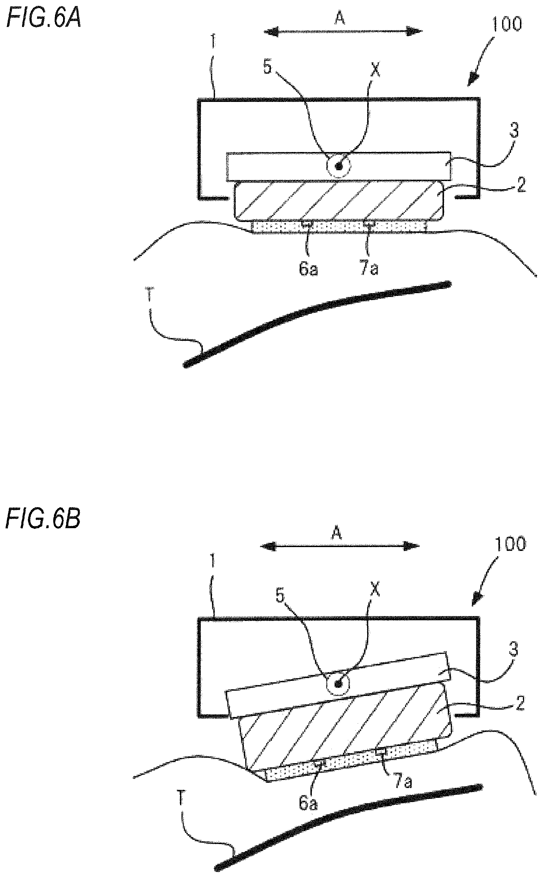

Figs. 6A and 6B are schematic views for illustrating operations of the biometric information measurement device of the embodiment. Figs. 6A and 6B illustrate the pulse wave detection unit 100 attached to the left wrist of the measurement subject, as seen from the direction B.

As shown in Figs. 6A and 6B, the radius artery T and the pressing surface 6b may not be parallel with each other due to the pressing force, which is generated as the sensor unit 6 is pressed to the body surface, body composition of the measurement subject or the like. In this case, the control unit 12 rotates the support member 3 about the first axis X in the counterclockwise direction, as shown in Fig. 6B. Thereby, the pressing surface 6b of the sensor unit 6 is rotated about the first axis X in the counterclockwise direction, so that it is possible to enable the pressing surface 6b of the sensor unit 6 and the radius artery T to be parallel with each other. The pressing surface 6b and the radius artery T are enabled to be parallel with each other, so that it is possible to equally press the radius artery T. Therefore, it is possible to detect the pulse wave with the same condition between the element array 60 and the element array 70, so that it is possible to improve the detection accuracy of the pulse wave.

The control unit 12 drives the rotation mechanism 5 so that the radius artery T exists immediately below the element arrays 60, 70 of the sensor unit 6, and sets a state, in which the rotation mechanism 5 is driven so that the pressing surface 6b and the radius artery T are parallel with each other, as the pulse wave measurement state. Then, in a state where the sensor unit 6 is pressed to the body surface with an appropriate pressure in the pulse wave measurement state, the control unit stores the pulse wave detected by any pressure detection element of the sensor unit 6 and calculates the biometric information based on the stored pulse wave.

As described above, according to the biometric information measurement device of the embodiment, it is possible to improve the detection accuracy of the pulse wave by flexibly changing the pressing state of the sensor unit 6, which is used with being pressed to the body surface, to the body surface.

Also, as shown in Figs. 5A to 5C, the sensor unit 6 is enabled to roll rotate, so that it is possible to select a posture of the sensor unit 6 with which it is difficult to detect the pressure signal from the tissue harder than the artery, such as the radius TB, the tendon or the like by the pressure detection element of the sensor unit 6. Accordingly, it is possible to reduce a noise of the pressure signal, which is to be detected by the pressure detection element of the sensor unit 6, thereby improving the detection accuracy of the pulse wave.

Also, according to the biometric information measurement device of the embodiment, the rotation mechanism 5, the air bag 2 and the sensor unit 6 are arranged in this order in the pressing direction of the sensor unit 6 by the air bag 2. According to this configuration, since it is possible to increase a distance between the first axis X or the second axis Y, which is a rotary axis of the rotation mechanism 5, and the pressing surface 6b, it is possible to increase patterns of the pressing posture of the pressing surface 6b. As a result, it is possible to improve the detection accuracy of the pulse wave.

Also, when the pressing posture of the pressing surface 6b is changed, the state as shown in Fig. 5C is obtained, so that it is possible to implement the optimal pulse wave measurement state without detaching the biometric information measurement device.

The rotation mechanism 5 of the biometric information measurement device of the embodiment may be a mechanism configured to rotate the support member 3 about only one of the first axis X and the second axis Y, i.e., a mechanism configured to rotate the sensor unit 6 about only the first axis X or the second axis Y.

In the case where the support member 3 is configured to rotate about only the second axis Y (only the roll rotation is enabled), one of the element array 60 and the element array 70 is not necessarily required in the sensor unit 6. That is, the sensor unit 6 may be provided with at least one element array. When a plurality of element arrays is provided, it is possible to improve the detection accuracy of the pulse wave.

The above-disclosed embodiment should be considered in all respects to be illustrative and not restrictive. The scope of the present invention is represented by the appended claims rather than the foregoing description.

For example, in the above, the wrist attached-type biometric information measurement device configured to detect the pulse wave from the radius artery of the wrist has been described. However, the present invention can be also applied to a device configured to detect the pulse wave from the carotid artery or dorsal pedis artery.

The sensor unit 6 may have a configuration where three or more element arrays are arranged in the direction A.

### Industrial Applicability

The present invention is convenient and useful particularly in application to a blood pressure monitor or the like.

Although the present invention has been described with reference to the specific embodiment, the present invention is not limited to the embodiment, and various changes can be made without departing from the technical concept of the disclosed invention which is defined by the appended claims.

The present application is based on Japanese Patent Application No. 2016-089789 filed on April 27, 2016.

### Explanation of Reference Numerals

- 100: pulse wave detection unit
- 1: housing
- 2: air bag
- 3: support member
- 5: rotation mechanism
- 6: sensor unit
- 6a, 7a: pressure detection element
- 6b: pressing surface
- 60, 70: element array
- X: first axis
- Y: second axis
- 10: rotation drive unit
- 11: air bag drive unit
- 12: control unit
- 13: display unit
- 14: operation unit
- 15: memory
- TB: radius
- T: radius artery
- A, B: direction

## Claims

1. A pulse wave detector comprising:
a sensor unit (6) which includes an element array (60) including a plurality of pressure detection elements (6a) arranged in one direction (B);
a pressing part (2) which is configured to press the sensor unit (6) to a body surface of a living body in a state where the one direction (B) intersects with an extension direction of an artery (T) under the body surface;
a rotation mechanism (5) which is configured to rotate the sensor unit (6) about a second axis (Y) extending in a direction perpendicular to each of a pressing direction of the pressing part (2) and the one direction (B);
a rotation drive unit (10) which is configured to drive the rotation mechanism (5); and the pulse wave detector is configured by a control unit (12) which is configured to control the rotation drive unit (10) so as to control a rotating angle of the sensor unit (6),
**characterized in that**
the pressing part (2) functions as an air bag which is configured to press the sensor unit (6) to the body surface in accordance with an air amount introduced therein, and
the rotation mechanism (5), the pressing part (2) and the sensor unit (6) are arranged in this order in the pressing direction.

2. A biometric information measurement device comprising:
the pulse wave detector according to claim 1; and
a biometric information calculation unit (12) which is configured to calculate biometric information based on pulse waves detected by the pressure detection elements (6a) of the sensor unit (6).

## Patentansprüche

1. Pulswellendetektor, umfassend:
eine Sensoreinheit (6), die eine Elementanordnung (60) aufweist, die mehrere Druckerfassungselemente (6a) aufweist, die in einer Richtung (B) angeordnet sind;
ein Druckteil (2), das eingerichtet ist, um die Sensoreinheit (6) an eine Körperoberfläche eines lebenden Körpers in einem Zustand zu drücken, in dem die eine Richtung (B) sich mit einer Ausdehnungsrichtung einer Arterie (T) unter der Körperoberfläche schneidet;
einen Drehmechanismus (5), der eingerichtet ist, um die Sensoreinheit (6) um eine zweite Achse (Y) zu drehen, die sich in einer Richtung senkrecht zu sowohl einer Druckrichtung des Druckteils (2) als auch der einen Richtung (B) erstreckt;
eine Drehantriebseinheit (10), die eingerichtet ist, um den Drehmechanismus (5) anzutreiben; und der Pulswellendetektor ist von einer Steuereinheit (12) gebildet, die eingerichtet ist, um die Drehantriebseinheit (10) zu steuern, um einen Drehwinkel der Sensoreinheit (6) zu steuern,
**dadurch gekennzeichnet, dass**
das Druckteil (2) als Luftsack dient, der eingerichtet ist, um die Sensoreinheit (6) entsprechend einer Luftmenge, die in sie eingeführt wird, an die Körperoberfläche zu drücken, und
der Drehmechanismus (5), das Druckteil (2) und die Sensoreinheit (6) in dieser Reihenfolge in der Druckrichtung angeordnet sind.

2. Biometrische-Informationen-Messvorrichtung, umfassend:
den Pulswellendetektor nach Anspruch 1; und
eine Berechnungseinheit (12) für biometrische Informationen, die eingerichtet ist, um biometrische Informationen basierend auf Pulswellen zu berechnen, die durch die Druckerfassungselemente (6a) der Sensoreinheit (6) erfasst werden.

## Revendications

1. Détecteur d'ondes de pouls comprenant :
une unité de détection (6) qui comporte une matrice d'éléments (60) comportant une pluralité d'éléments de détection de pression (6a) agencés dans une direction (B) ;
une partie de pression (2) qui est conçue pour presser l'unité de détection (6) contre une surface du corps d'un corps vivant, dans un état dans lequel ladite direction (B) croise une direction d'extension d'une artère (T) sous la surface du corps ;
un mécanisme de rotation (5) qui est conçu pour faire tourner l'unité de détection (6) autour d'un second axe (Y) s'étendant dans une direction perpendiculaire à une direction de pression de la partie de pression (2) et à ladite direction (B) ;
une unité d'entraînement en rotation (10) qui est conçue pour entraîner le mécanisme de rotation (5) ; et le détecteur d'ondes de pouls est configuré par une unité de commande (12) qui est conçue pour commander l'unité d'entraînement en rotation (10) de façon à commander un angle de rotation de l'unité de détection (6),
**caractérisé en ce que**
la partie de pression (2) fonctionne comme un coussin d'air qui est conçu pour presser l'unité de détection (6) contre la surface du corps conformément à une quantité d'air introduite dans celui-ci, et
le mécanisme de rotation (5), la partie de pression (2) et l'unité de détection (6) sont agencés dans cet ordre dans la direction de pression.

2. Dispositif de mesure d'informations biométriques comprenant :
le détecteur d'ondes de pouls selon la revendication 1 ; et
une unité de calcul d'informations biométriques (12) qui est conçue pour calculer des informations biométriques sur la base d'ondes de pouls détectées par les éléments de détection de pression (6a) de l'unité de détection (6).
